# EUROPEAN PATENT SPECIFICATION

(11) **EP 2 882 437 B1**
(45) Date of publication and mention of the grant of the patent: **14.12.2016**
(21) Application number: 13752573.9
(22) Date of filing: 05.08.2013
(51) Int. Cl.: A61K 31/4439, A61K 31/404, A61K 31/506, A61K 31/519, A61P 35/02

(54) **CASEIN KINASE 1 INHIBITORS FOR THE TREATMENT OF B-CELL CHRONIC LYMPHOCYTIC LEUKEMIA**
HEMMER DER CASEIN KINASE 1 ZUR BEHANDLUNG VON CHRONISCHER LYMPHOCYTISCHER B-ZELLEN-LEUKÄMIE
INHIBITEURS DE CASÉINE KINASE 1 POUR LE TRAITEMENT DE LEUCÉMIE LYMPHOÏDE CHRONIQUE À LYMPHOCYTES B

(30) Priority: 08.08.2012 CZ 20120538
(43) Date of publication of application: 17.06.2015
(73) Proprietor: Masarykova Univerzita, 601 77 Brno (CZ)
(72) Inventor: BRYJA, Vitezslav, 621 00 Brno (CZ); KAUCKA, Marketa, 500 12 Hradec Kralove (CZ); PLEVOVA, Karla, 756 22 Hostalkova u Vsetina (CZ); PAVLOVA, Sarka, 625 00 Brno (CZ); POSPISILOVA, Sarka, 616 00 Brno (CZ); KOZUBIK, Alois, 623 00 Brno (CZ)
(74) Representative: Hartvichova, Katerina
(86) International application number: PCT/CZ2013/000090
(87) International publication number: WO 2014/023271

(56) References cited:
- US-A1- 2010 179 154
- MATUTES ET AL: "Chronic lymphocytic leukaemia", 20040601, vol. 32, no. 6, June 2004 (2004-06), pages 72-74, XP005770001,
- NOWELL P ET AL: "Cytogenetics of chronic B-cell and T-cell leukemia", CANCER GENETICS AND CYTOGENETICS, ELSEVIER SCIENCE PUBLISHING, NEW YORK, NY, US, vol. 1, no. 3, March 1980 (1980-03), pages 273-280, XP023450825, ISSN: 0165-4608, DOI: 10.1016/0165-4608(80)90024-2 [retrieved on 1980-03-01]
- YANG WAN SEOK ET AL: "Inhibition of casein kinase 1-epsilon induces cancer-cell-selective, PERIOD2-dependent growth arrest", GENOME BIOLOGY, BIOMED CENTRAL LTD., LONDON, GB, vol. 9, no. 6, 2 June 2008 (2008-06-02), page R92, XP021041640, ISSN: 1465-6906
- GUTHOFF I ET AL: "THE CASEIN KINASE 1 DELTA (CK1 DELTA) SPECIFIC INHIBITOR IC261 IMPINGES GROWTH OF PANCREATIC TUMOR CELLS AND THE EXPRESSION OF CK1DELTA IN HEALTHY YOUNG ADULT BALB/C MICE", EUROPEAN JOURNAL OF CANCER. SUPPLEMENT, PERGAMON, OXFORD, GB, vol. 1, no. 5, 21 September 2003 (2003-09-21), page S298, XP009048898, ISSN: 1359-6349, DOI: 10.1016/S1359-6349(03)91021-4

## Description

### Technical field

The invention relates to inhibitors of casein kinase 1 for use in the treatment of B-cell chronic lymphocytic leukemia.

### Background Art

B-cell chronic lymphocytic leukemia (CLL) is clinically a very heterogeneous disease with an unclear pathogenesis. The current findings indicate that the cause of CLL is a monoclonal expansion of B-lymphocytes that then accumulate in both peripheral blood and lymphatic organs, which results in clinical complications such as hypertrophy of organs, reduced function of the immune system, anemia and others. It is believed that the disease evolves as a result of the defects in apoptosis and changes in the migration of B-lymphocytes.

CLL is characterized by the accumulation of dysfunctional malignant monoclonal B-lymphocytes in the blood and their migration to lymphatic nodes, liver, spleen, and bone marrow. The clinical progression of the disease then depends on the interaction of these dysfunctional cells with their immediate environment (micro-environment). This interaction then leads to uncontrolled proliferation of tumor cells.

CLL is an incurable disease. The patients are regularly monitored and the treatment is commenced only at a significant progression of clinical symptoms. The standard treatment is a combination of chemo- and immuno-therapy (FCR; fludarabine+cyclophosphamide and rituximab, which is a monoclonal antibody against surface receptor of B-lymphocytes), which leads to retreat of symptoms in majority of patients during the first treatment. Typically, however, the disease returns again after the first cycle of therapy and further therapy is often less effective. Moreover, the suitability of FCR regimen is very limited in patients with aggressive CLL associated with the deletion/mutation of the p53 tumor suppressor.

At present, huge effort is invested into the development and testing of drugs targeting cellular pathways involved in the interaction of CLL cells with their microenvironment in lymphatic organs. The medicaments are targeted in particular to the pathways activated upon interaction of the cells with antigen (signaling through the B-cell receptor, BCR), between adhesion molecules, and upon interaction of cellular receptors with the so-called chemotactic cytokines - chemokines that control the migration of the cells of the immune system in the body and inside the lymphatic organs. According to the first results, some of these substances show promising results even in patients who do not respond to FCR (Wayach JA et al., Blood 2012 Jun 12; Honigberg LA et al., PNAS 2010 Jul 20; 107(29): 13075-80; Herman SE et al., Blood. 2011 Jun 9; 117(23): 6287-96; Herman SE et al., Blood. 2010 Sep 23; 116(12): 2078-88). So far, however, there is no therapy routinely used in clinic, which targets primary causes of the disease.

### Disclosure of the Invention

The object of the present invention are inhibitors of casein kinase 1 (CK1) for use in the treatment of B-cell chronic lymphocytic leukemia (CLL).

The subject of the present invention is an inhibitor of casein kinase 1 selected from the group comprising D4476, PF670462, IC261, PF 4800567 for use in the treatment of B-cell chronic lymphocytic leukemia.

The CK1 inhibition prevents the migration of CLL cells in a chemokine gradient, thus preventing the cells from getting to the microenvironment that activates them. The CK1 inhibitors are capable of efficiently inhibiting the migration of leukemia cells to lymphatic organs. By this mechamism they prevent both the interaction of leukemia cells with the micro-environment of these organs leading to impaired functionality of these organs and development of CLL.

In one embodiment of the invention, the CK1 inhibitor is D4476 (CAS No: 301836-43-1):

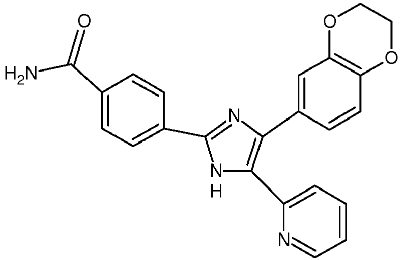

In another embodiment of the invention, the CK1 inhibitor is PF670462 (CAS No: 950912-80-8):

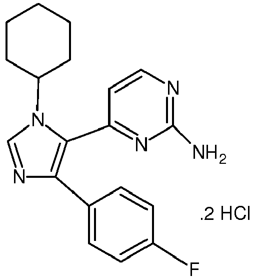

In a further embodiment of the invention, the CK1 inhibitor is IC261 (CAS No: 186611-52-9):

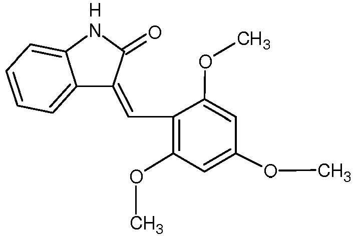

In yet another embodiment of the invention, the CK1 inhibitor is PF 4800567 (CAS No: 1188296-52-7):

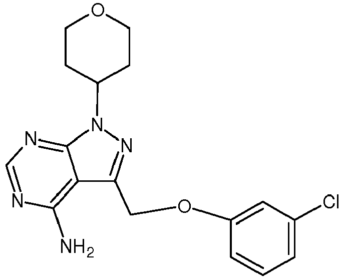

The subject of the present invention is further a medicament for use in the treatment of B-cell chronic lymphocytic leukemia, containing at least one casein kinase 1 inhibitor, selected from the group comprising D4476, PF670462, IC261, and PF 4800567.

### Brief Description of Figures

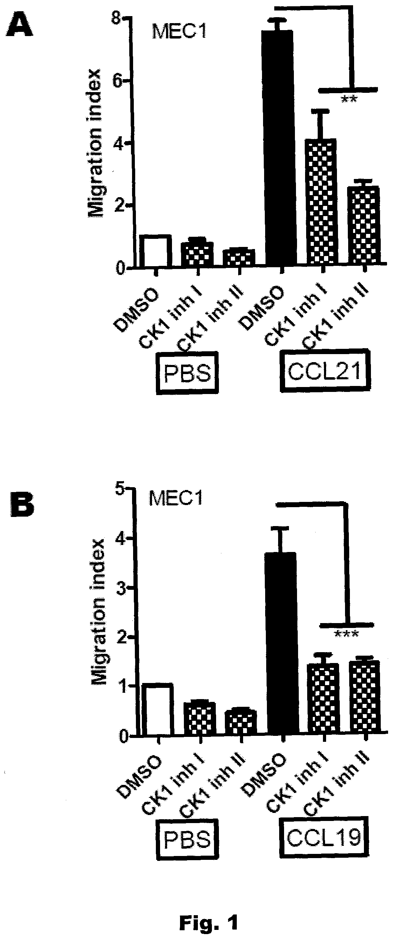

Figure 1: Migration of MEC1 cells which represents an in-vitro CLL model was investigated in the presence of chemokines CCL19 and CCL21 (200 ng/ml) using Transwell migration chamber. The rate of migration is expressed as a migration index. The MEC1 migration triggered by the chemokines CCL21 and CCL19 is blocked by CK1 inhibitors - D4476 (CK1 inh. I; 100 µM) and PF670462 (CK1 inh. II; 50 µM). The values in the Figs. 1A and 1B represent the average and the standard error. Statistical significance was tested using One-way ANOVA and Tukey post-hoc test (*p<0,05, **p<0,01, ***p<0,001).

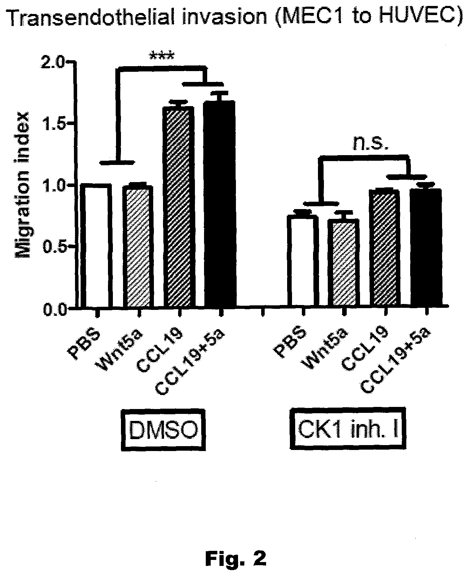

Figure 2: Transendothelial invasiveness of the MEC1 cells was tested in the Transwell migration chamber in an arrangement where the upper chamber was covered with one layer of HUVEC endothelial cells. The rate of invasiveness (determined as the migration index) of the MEC1 cells was analyzed in the presence of the chemokine CCL19, Wnt5a, or in combination of both substances. The positive effect of CCL19 to the transendothelial migration of MEC1 was inhibited by addition of D4476 (CK1 inh. I). Statistical significance was tested using One-way ANOVA test and Tukey post-hoc test (***p<0,001). Each experiment was independently repeated three-times.

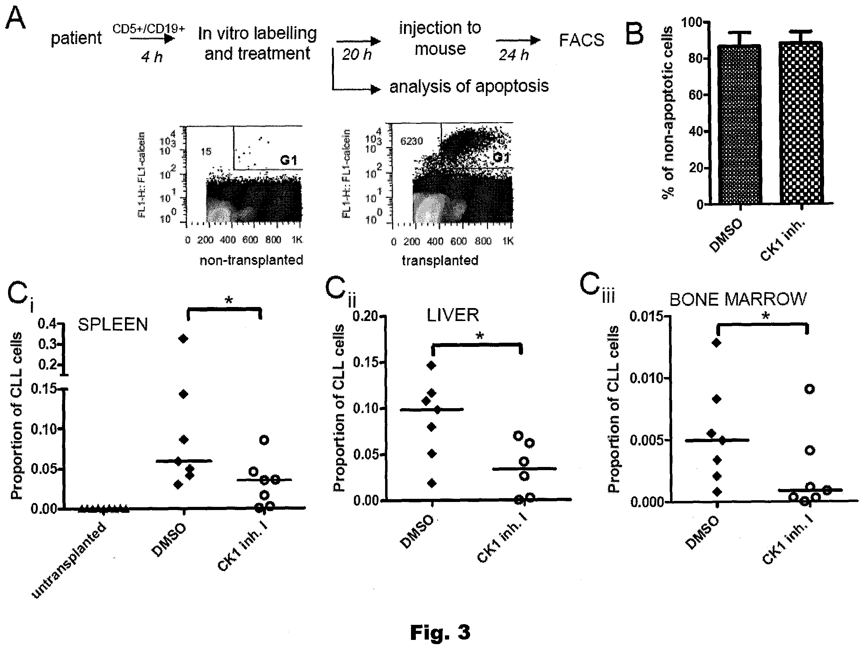

Figure 3: CK1 controls the migration of CLL cells in vivo. (A) Layout of the experiment: CLL cells were obtained by purification from the samples of fresh blood from patients, stimulated and stained in vitro using green calcein. They were subsequently intraperitoneally injected to NSG mice. After 24 hours the murine tissues were examined for the presence of calcein-positive CLL cells using flow-cytometry (refer to the G1 cut-out in the typical dot plot distribution). (B) The rate of apoptosis in primary CLL cells after adding DMSO/D4476 (CK1 inh. I) was determined by the analysis of membrane potential of mitochondria using flow-cytometry while using tetramethylrhodamine ethyl ester (TMRE) stained with green calcein dye. The charts show that the stimulation by casein kinase 1 inhibitors does not alter the rate of apoptosis (mean + standard error). (C) Effects of the inhibition of CK1 on infiltration of murine tissue by the CLL cells. The charts show the ratio of the CLL cells (the quantity of positive cells / total quantity of cells in the organ) treated by DMSO or D4476 (CK1 inh. I) that were obtained from the spleen (Cᵢ), liver (Cᵢᵢ), and bone marrow (Cᵢᵢᵢ) of transplanted mice. One symbol in the chart corresponds to one patient. * p < 0.05 - Wilcoxon pair t-test.

### Examples of carrying out the Invention

### Example 1: Effect of CK1 inhibitors to the migration of CLL cells

In this example, the MEC1 cell line was used, which is a well-defined cell line obtained from a CLL patient in a pro-lymphocytic transformation. The MEC1 line is used as a transplantation model of CLL. MEC1 were obtained from the DSMZ collection (Braunschweig, Germany) and they were cultivated in RPMI1640 supplemented with 10% FBS and antibiotics at 37 °C and 5 % of CO₂.

Into the upper insert of HTS Transwell^{®} 96-well plates (Coming Incorporated, Mexico) with polycarbonate membranes with the pore size of 5.0 µm, 0.5 x 10⁶ MEC1 cells were seeded. The cells were treated overnight with D4476 (CK1 inhibitor, 100 µM, Calbiochem, San Diego, CA, USA) in DMSO, or PF670462 (CK1 inhibitor, 50 µM; Tocris Biosciences, Ellisville, Missouri, USA) in DMSO, or DMSO (dimethyl sulfoxide; control - solvent of the inhibitors above, used in the same amount as in the experimental wells), and incubated in the complete medium (including 10% FCS) at 37 °C and 5 % of CO₂. The migration to chemokine was then analyzed using the Coulter Counter instrument (model F_{N}, Coulter Electronics, Florida, USA). The migration index was calculated as the number of cells (treated or non-treated) migrating to the chemokine divided by the number of cells migrating only to the control medium.

In this example, inhibition of CK1ε kinase is tested, which is required for PCP signalization due to its role in the phosphorylation of the Dvl protein. The inhibition of CK1ε using D4476 (CK1 inh. I) blocked the chemotactic response of MEC1 cells to CCL21 and CCL19 (Figure 1), which shows that CK1ε is necessary for the migration of CLL to CCL21 and CCL19. To eliminate the effects that are non-specific for CK1ε, we repeated the same procedure with a well-defined PF670462 inhibitor specific for CK1δ and CK1ε with almost the same results.

### Statistical methods:

T-test or Mann-Whitney test were used for statistical evaluation to assess the difference between two continual variables, one-way ANOVA test (followed by the Tukey's post-hoc test) to evaluate the difference between more than two variables. The standard level of statistical significance was p=0.05. All statistical evaluations were performed using the GraphPad Prism 5 (GraphPad Software Inc., La Jolla, California).

### Example 2: Effect of a CK1 inhibitor on the chemokine-controlled transendothelial invasion of CLL cells

In the complex environment of the human body, cells react to the attractants and have to pass through extra-cellular matrix or endothelial barrier, which is a process known as invasion. It was tested whether the CK1 inhibitors affect the invasion through a layer of human umbilical vein endothelial cell (HUVEC).

Into the upper insert of HTS Transwell^{®} 96-well plates (Corning Incorporated, Mexico) with polycarbonate membranes with the pore size of 5.0 µm, covered with HUVEC (human umbilical vein endothelial cells monolayer), 0.5 x 10⁶ MEC1 cells were seeded. The cells were treated overnight with D4476 (CK1 inhibitor, 100 µM, Calbiochem, San Diego, CA, USA) in DMSO, or PF670462 (CK1 inhibitor, 50 µM; Tocris Biosciences, Ellisville, Missouri, USA) in DMSO, or DMSO (dimethyl sulfoxide; control - solvent of the inhibitors above, used in the same amount as in the experimental wells), and incubated in the complete medium (including 10% FCS) at 37 °C and 5 % of CO₂. The migration to chemokine was then analyzed using the Coulter Counter instrument (model F_{N}, Coulter Electronics, Florida, USA). The migration index was calculated as the number of cells (treated or non-treated) migrating to the chemokine divided by the number of cells migrating only to the control medium.

First, the ability of CXCL12, CCL19, and CCL21 to induce the invasion of MEC1 cells in the Transwell wells coated with HUVEC was characterized. Of these three tested chemokines, only CCL19 was able to induce the transendothelial migration of the MEC1 cells. This response was not increased by Wnt5a, but it was significantly decreased by the CK1 inhibitor (Figure 2).

### Example 3: Inhibition of migration of CLL cells in tissues in vivo by CK1 inhibitors

NOD SCID IL2R gamma null (NSG) mice obtained from The Jackson Laboratory (Bar Harbor, ME, USA) were held in special pathogen-free conditions. These mice lack adult T-cells, B-cells and functional NK-cells. Non-irradiated mice (at the age of 8 to 16 weeks) were used for transplantation; the experiments were performed in accordance with legal provisions of the Czech Republic.

Primary B-cells from untreated patients with CLL were separated by gradient centrifugation followed by depletion of non-B-cells (RosetteSep® B Cell Enrichment Kit and Human CD3+ Depletion Kit; StemCell Technologies, Vancouver, Canada; or MACS B cell Isolation Kit II; Miltenyi Biotec, Bergisch Gladbach, Germany) according to the manufacturer's instructions. Evaluation of the expression profile of CD5 and CD19 of purified cells was performed using flow cytometry (three-color panel: CD45-TRI-COLOR, MHCD45065, Invitrogen, CD5-FITC, A08932, CD19-PE, A07769, Beckman Coulter). Samples with the purity higher than 95 % of B-cells were used for further analyses.

Freshly isolated human CLL lymphocytes (25 x 10⁶ from the patient) were pre-treated for 20 hours with a CK1 inhibitor (100 µM, D4476, Calbiochem), and dimethylsulfoxide (DMSO, Sigma-Aldrich) as control. The cells were then stained with Calcein AM (200 nM, Invitrogen) for 45 minutes and washed twice with sterile PBS. An aliquot of the pre-treated and Calcein AM stained cells (5 x 10⁶) was used for FACS analysis (FACScalibur) to determine the effects on survival using the decrease of themitochondrial membrane potential as the readout (2 µM TMRE (tetramethylrhodamine ethyl ester), Invitrogen, 20 minutes of staining at the laboratory temperature). The transplantation to mice was performed by intra-peritoneal injection of 20 x 10⁶ pre-treated and Calcein AM stained human CLL lymphocytes in 120 µl of sterile PBS. Samples of spleen, liver, and femoral bone marrow of mice were taken 24 hours after the injection and analyzed. Migration of calcein-positive CLL cells to these tissues was determined by FACS analysis (FACScalibur).

In this experiment, the rate of apoptosis of treated cells was determined by flow cytometry of the mitochondrial membrane potential using TMRE staining in combination with green calcein staining marking the living cells. The double-positive cells were considered as living and non-apoptotic.

The primary CLL cells in the NSG mice actively migrate into the corresponding tissues and they can be detected especially in the spleen, liver and bone marrow. The treatment by the CK1 inhibitor D4476 reduced the migration to the spleen, liver and bone marrow, without an increase of cell apoptosis being detected (Figure 3). This experiment shows that the inhibition of CK1 reduces the ability of the CLL cells to migrate and colonize the tissues of mice.

## Claims

1. An inhibitor of casein kinase 1 selected from the group comprising D4476, PF670462, IC261, PF 4800567 for use in the treatment of B-cell chronic lymphocytic leukemia.

2. A medicament for use in the treatment of B-cell chronic lymphocytic leukemia, **characterized in that** it contains an inhibitor of casein kinase 1 selected from the group comprising compounds D4476, PF670462, IC261, PF 4800567.

## Patentansprüche

1. Inhibitor der Casein-Kinase 1 ausgewählt aus der Gruppe D4476, PF670462, IC261, PF 4800567 zur Verwendung bei der Behandlung von B-Zell-chronischer lymphatischer Leukämie.

2. Medikament zur Verwendung bei der Behandlung von B-Zell-chronischer lymphatischer Leukämie, **dadurch gekennzeichnet, dass** es einen Inhibitor der Casein-Kinase 1 enthält, ausgewählt aus der Gruppe der Verbindungen D4476, PF670462, IC261, PF 4800567.

## Revendications

1. Un inhibiteur de caséine kinase 1 choisi dans le groupe comprenant D4476, PF670462, IC261, PF 4800567 pour une utilisation dans le traitement de la leucémie lymphocytaire chronique à cellules B.

2. Médicament destiné à être utilisé dans le traitement de la leucémie lymphocytaire chronique à cellules B, **caractérisé en ce qu'**il contient un inhibiteur de caséine kinase 1 choisi dans le groupe comprenant les composés D4476, PF670462, IC261, PF 4800567.
